# EUROPEAN PATENT SPECIFICATION

(11) **EP 1 465 505 B1**
(45) Date of publication and mention of the grant of the patent: **18.11.2015**
(21) Application number: 02799071.2
(22) Date of filing: 23.12.2002
(51) Int. Cl.: A23L 1/308, A23K 1/16, A61P 37/04, A61P 1/00

(54) **STIMULATION OF THE IMMUNE SYSTEM WITH POLYDEXTROSE**
STIMULATION DES IMMUNSYSTEMS MIT POLYDEXTROSE
STIMULATION DU SYSTEME IMMUNITAIRE AVEC DU POLYDEXTROSE

(30) Priority: 15.01.2002 FI 20020078
(43) Date of publication of application: 13.10.2004
(73) Proprietor: DuPont Nutrition Biosciences ApS, 1001 Copenhagen K. (DK)
(72) Inventor: RAUTONEN, Nina, FIN-02170 ESPOO (FI); APAJALAHTI, Juha, FIN-00670 HELSINKI (FI); TIIHONEN, Kirsti, 00160 Helsinki (FI)
(86) International application number: PCT/EP2002/014719
(87) International publication number: WO 2003/059333

(56) References cited:
- EP-A- 0 593 774
- EP-A- 0 788 744
- EP-A- 0 821 885
- ZHONG JIE ET AL.: "Studies on the effects of polydextrose intake on physiologic funcitons in Chinese people" AMERICAN JOURNAL OF CLINICAL NUTRITION, vol. 72, no. 6, 1 December 2000 (2000-12-01), pages 1503-1509, XP002238576 BETHESDA,MD, US ISSN: 0002-9165 cited in the application
- TOMOTARI MITSUOKA: "Intestinal flora and human health" ASIA PACIFIC JOURNAL OF CLINICAL NUTRITION, vol. 5, no. 1, 1996, pages 2-9, XP002238577
- EDWARDS, W P : "Not naughty, but nice" CHEMISTRY IN BRITAIN , vol. 33, no. 11, 1997, pages 50-52, XP008020435 GREAT BARDFIELD, GB

## Description

### FIELD OF THE INVENTION

This invention relates to the use of polydextrose for stimulating the immune response in the gastrointestinal tract of a mammal. Furthermore, but not exclusively, the invention relates to an improved functional food or feed composition comprising polydextrose and having a local immunostimulating effect in the intestinal tract. In a preferred embodiment of the invention polydextrose is combined with a polyol, which enhances the immunostimulating effect. In a further beneficial aspect of the invention polydextrose is used in combination with a polyol for improving the intestinal health of a mammal by reducing the factors which increase the risk of colon cancer.

### BACKGROUND OF THE INVENTION

The health and well being of people and animals can be positively or negatively influenced by the micro-organisms which pass and inhabit the gastrointestinal tract. These micro-organisms affect the physiological condition of the host through the production of toxins, metabolic byproducts, short chain fatty acids, and the like.

The intestinal lumen comprises a large interface with the environment. The primary function of the small intestine is to absorb nutrients from the food. In order to allow maximal nutrient absorption, the contact area needs to be large. The mucosal membrane of the whole human intestine covers over 200 m² of area. Over 30 tons of food and approximately the same amount of drinks pass through the intestine during a lifetime. However, in addition to dietary compounds, the mucosal surface is exposed to various bacteria, viruses, parasites and fungi. Due to the monolayered epithelium covering the lumen, it is also an attractive gate for pathogens to the body. The state of the intestinal tract is a significant factor in many illnesses (e.g. infections, allergies and cancer).

Many pathogenic bacteria and viruses initially gain entry into the body by crossing the cellular linings (epithelia) of the gastrointestinal, respiratory, or genital tracts. A specialized class of antibodies, immunoglobulin A (IgA) antibodies, protects these surfaces. IgA antibodies are dimeric or polymeric molecules produced by cells located in the tissues under the epithelial surfaces. They are transported by epithelial cells into mucosal secretions, where they cross-link or coat pathogens that have not yet entered the body, preventing the pathogens from contacting and adhering to epithelial cells. Thus, IgA antibodies operate on pathogens that are outside the body, and they protect by preventing entry into the body across epithelial surfaces molecules. Moreover, by binding to some dietary or other environmental compounds the antibodies can also prevent unnecessary inflammatory reactions in the gut. Over 80 % of the antibody producing cells in the body are located in the gut. This reflects both the importance of IgA function in the gut health as well as the large surface that needs to be protected.

The mammalian large intestine contains a substantial and diverse population of bacteria that is important to mammalian health. The beneficial microflora in the gut is able to salvage energy for the host through bacterial fermentation of undigested carbohydrates and proteins to provide short-chain fatty acids, which are then absorbed. The beneficial genera, *Bifidobacterium* and *Lactobacillus,* both of which are saccharolytic, are thought to create conditions unfavourable for growth of potentially pathogenic species. Hence, bifidobacteria and lactobacillus are ingredients in various probiotic products, which are used to elevate the number of said microbes in the gut. On the other hand, various kinds of food and drink compositions, which include non-absorbable carbohydrates (oligosaccharides and polysaccharides) have been developed. These are called prebiotics and they are thought to stabilize the intestinal microbial balance in-favour to the beneficial microbes. Furthermore, dietary fiber products have been used to treat constipation and to promote laxation.

Interaction of resident intestinal microbes and the immune system is still largely unknown. It is known that after birth the intestinal immune system developes only poorly without microbes. Hence, it has been shown that the numbers of CD4+ and CD8+ immune cells and IgA producing cells, as well as cytotoxic activity are decreased, or almost absent, in germfree animals (Rothkötter et al., Pediatr. Res. 1991 Mar, 29(3):237-42).

Epidemiological studies have shown that the intake of cereals or non-starch polysaccharide fiber reduces the risk of developing colon cancer. But also controversial results have been recently reported on the role of dietary fibres in colon carcinogenesis. The fibers might have very different kinds of effects on the factors which enhance carcinogenesis. All fibers do not necessarily reduce the risk of colon cancer. A comparative study with cereal diets (wheat, oat, rye) and inulin has showed, that an inulin diet increases the protein kinase C (PKC) activity and PKC β2 protein level in the distal colon. Since, PKC β2 mediates colonic cell proliferation, and overexpression of PKC β2 has been found in colon cancer cell lines, PKC β2 expression and activity are thought to enhance colon carcinogenesis. (A-M. Pajari et al., British J. Nutrition, 8, pp. 635-643, 2000*).* Also, a comparative study with Min mice (multiple intestinal neoplasia cancer model) fed with various diets, showed no tumor-preventing effects when mice were fed with inulin or beef meat, but in the mice fed with rye bran or wheat bran, significant reduction of tumorigenesis was detected (M. Mutanen et al. Carcinogenesis, Vol. 21, No. 6, pp. 1167-1173, 2000*).* Another comparative study with a low cholesterol diet, a high cholesterol diet, and a high cholesterol diet supplemented by polydextrose showed that the diet with polydextrose significantly lowered fecal pH, and reduced the production of some carcinogens, like indole and p-cresol. (Endo, K. et al. Bifidobacteria Microflora, Vol. 10 (1),53-64, 1991).

Polydextrose is a polysaccharide synthesized by random polymerisation of glucose, sorbitol and and a suitable acid catalyst at high temperature and partial vacuum. The term "polydextrose" is defined in greater detail later in this text. Polydextrose is widely used in various kinds of food products as a bulking agent and as a low- energy ingredient, replacing sugar and partially fat. Polydextrose is not digested or absorbed in the small intestine and a large portion is excreted in the feces. Polydextrose has been incorporated into a wide range of foods including baked goods, beverages, confectionary and frozen dairy desserts. The beneficial effects of polydextrose on the intestinal tract have been described in Am. J. Clin. Nutr. 72, pp.1503-1509, 2000 *and in* Bifidobacteria Microflora, Vol. 10 (1), pp.53-64, 1991*.* US 5,437,880 (Otsuka Pharma) describes a health drink containing polydextrose; JP 2072842 (Showa Sangyo) describes a drink and food containing polydextrose as a dietary fiber, and EP 821885 (Raffinerie Tirlemontoise) describes a dairy powder containing polydextrose for promoting the function of the intestine.

The beneficial effects of polydextrose on the intestinal tract are well-known, but the inventors of the present invention have now surprisingly found, that daily intake of polydextrose very efficiently stimulates the immune system in the gut of a mammal. This effect has been demonstrated by very significantly elevated levels of IgA. At the same time it was found that polydextrose causes no increase in pro-inflammatory markers, i.e. there is no increased risk of inflammation of the gut.

Furthermore, the inventors have found, that the immunostimulating effect of polydextrose on the intestinal tract was significantly improved when the dosage of polydextrose was further fortified with polyol. The immunostimulating effect of polydextrose alone or in combination with a polyol is significantly improved when these compounds are included in the daily diet over a long time.

In addition to the synergistically benefial effects of polydextrose and polyol on the immune system in the gut, the inventors have also found that the use of polydextrose in combination with a polyol very significantly reduces the amount of biogenic amines in the colon, thus decreasing the substances which are end products of bacterial metabolism of proteins. Some biogenic amines have toxic properties and are known to cause higher risk of colon cancer. Furthermore, the inventors have found that when polydextrose is used in combination with a polyol, no significant laxative effect is provided even though the polyol on its own might have caused a laxative reaction.

Polyols are sugar-free sweeteners, also called sugar alcohols because part of their structure chemically resembles sugar and part is similar to alcohols. Other terms used are polyhydric alcohols and polyalcohols. Those currently used in foods in the U.S. are erythritol, hydrogenated starch hydrolysates (including maltitol syrups), isomalt, lactitol, maltitol, mannitol, sorbitol and xylitol. They are used as sugar substitutes in a wide range of products, including chewing gums, candies, ice cream, baked goods and fruit spreads. They are also used in toothpastes, mouthwashes, breath mints and pharmaceuticals such as cough syrups or drops and throat lozenges. Many polyols, like lactitol, sorbitol, xylitol and mannitol have in higher doses laxative effects. A lactitol-containing growth-stimulating feed and a lactitol-lactulose -containing feed for promoting growth and intestinal function of animals are described in U.S. Pat. No. 4,760,055 and EP 0 464 362, respectively (both assigned to Valio Meijeri). A pharmaceutical composition comprising living bacteria, salts and not adsorbable carbohydrates (e.g. polyols) for regenerating intestinal flora in diarrhea or dyspeptic syndrome is disclosed in WO 00/54788 A1 (Italmed S.N.C.)

Examples of the combined use of polydextrose and a polyol include improvements in the field of sugar products for food processing, as disclosed in WO 95/26644 A1 (Worlee Sweet), and in WO 98/34500 A1 (Purac Biochem B.V.), and low-calorie chocolate products, which contain sugar alcohols, and polydextrose as a fiber as disclosed in U.S. Pat. No. 5,629,040 (Lotte Co Ltd.). A dietetic product "milk jam" free of saccharose and sugar and which includes polyol (lactitol and sorbitol) and polydextrose is disclosed in a patent application in Uruguayan Ser. No. 26,096 (Gabriel Julio Flangini Morales). Reduced-calorie frozen desserts containing polydextrose and polyols are described in U.S. Pat No. 5,527,554 (Xyrofin Oy), reduced calorie chocolate composition containing polydextrose, maltitol and lactitol are described in PCT/US00/0024 (Xyrofin Oy) and a sugar-free boiled confectionery comprising polydextrose and lactitol is described in EP 452262, (Xyrofin Oy). A reduced calorie sugar free sweetening composition for a cooked confection product comprising xylitol and polydextrose is disclosed in U.S. Pat. No. 5,098,730 (Danisco Cultor). Another low-calorie confectionary comprising a polyol and polydextrose is described in JP 61254148 (Ishiwatari K.). The use of polydextrose and xylitol as a satiety agent is disclosed in background art U.S. Patent Application Ser. No. 60/282866. Tomotari Mitsuoka in Asia Pacific Journal of Clinical Nutrition, vol5, No. 1, 1996 pages 2-9, notes that polydextrose has a benefitial effect on the intestinal environment through changing the balance of the intestinal flora.

None of the above mentioned documents indicate any effect of polydextrose on the immune system of the gastrointestinal tract.

Most functional food products (like prebiotics and probiotics) targeted to the gastrointestinal tract have been until now focusing on modulation of the intestinal motility function and the absorbance capacity, as well as on the modulation of the microflora balance in the intestinal tract, which plays an important role in combating against pathogens.

It is well known that the immunological state of a patient could be influenced by the diet. Many attempts have been made to develop and improve immunostimulating preparations, like various types of food and drink products, in which the active ingredient has varied from different kinds and amounts of oligosaccharides, fatty acids, vitamins, minerals, trace elements and herbal extracts, and compositions thereof. These products have been developed to improve the immunological state in a patient generally, rather than locally in the intestine.

However, there is a need to develop convenient and effective methods which specifically support the local immune system in the intestinal tract, and which are useful for promoting the immune system of the intestine in a prophylactic manner and which are also useful for patients suffering from inflammation in the gastrointestinal tract, and for those patients suffering from impaired immune system especially in the gastrointestinal tract. Especially desirable would be food or diet supplement products, which allow a long-term safe administration of substances, which specifically stimulate the immune system in the gut and protects the intestinal tract against pathogenic microflora.

Such safe, tasty, and ready-to-eat products would be most practicable also in a prophylactic use by strengthening the immune system in the intestinal tract of healthy subjects, thus facilitating prevention of severe inflammation in a case of sudden exposition to pathogens, which often occurs e.g. when travelling in countries with less hygiene with food handling.

The present invention meets that need by providing a method for stimulation of the intestinal immune system a mammal by the use of polydextrose. The present invention overcomes deficiencies of the prior art, by providing a method for improved management and prevention of enteric diseases combining the already known beneficial effects of the use of polydextrose with improved immunological capacities of a mammalian body.

The present inventors have found that polydextrose provides immunomodulating shift in the microbial community. Thus, polydextrose can be used to normalize the gut microbial community and to enhance microbes with positive effects on the gut immune system.

Furthermore, the present invention also provides a method for potentiation of the immunostimulating effect of polydextrose in the intestinal tract by combining polydextrose with a polyol providing a synergistical improvement of the effect provided by polydextrose alone.

In addition to the immunostimulation, the inventors of the present application unexpectedly found that polydextrose, in combination with polyols, further can be used in providing a significant reduction of biogenic amines in the intestine, specifically in the caecum. The biogenic amines are known to cause higher risk of colon cancer. Excessive accumulation of biogenic amines, putrescine, spermidine and spermine favour malignant transformation of cells: enhanced ODC (omithine decarboxylase) activity is accompanied by the expression of several oncogenes (Wickström, Acta Ophthalmol. 70, pp. 506-514, 1991).

Hence, the present invention contributes to the overall health and well-being of the intestinal tract by providing a method, which not only effectively protects the gut against pathogens and dietary allergens, but also contributes to the prophylaxis of colon cancer.

### SUMMARY OF THE PRESENT INVENTION

Accordingly, it is an object of the present invention to provide methods and compositions for strengthening and improving the health condition of the mammalian gut. The objects of the invention are defined by the appended claims.

One aspect of the present invention is the use of polydextrose as an active ingredient in the preparation of a composition for stimulating the immune system of the gastrointestinal tract of a mammal, comprising mixing at least one pharmacologically acceptable carrier (or vehicle) with polydextrose, said polydextrose being effective in increasing the immunoglobulin A (IgA) concentration in the gut of said mammal.

Another aspect of the present invention is the use of polydextrose as an active ingredient in the preparation of a composition for stimulating the immune system of the gastrointestinal tract of a mammal, comprising mixing at least one polyol with polydextrose, said polyol being effective to synergistically increase the immunoglobulin A (IgA) concentration in the gut of said mammal.

A further aspect of the present invention relates to the use of polydextrose and a polyol for reducing the amount of the biogenic amines in the gut of a mammal. Accordingly, the present invention provides a method and a composition for diminishing the risk of a colon cancer by effective reduction of biogenic amines.

The methods of the present invention may take several embodiments. Additional objects, advantages and features of the various aspects of the present invention will become apparent from the following description of its preferred embodiments.

### BRIEF DESCRIPTION OF THE DRAWINGS

Figure 1. depicts graphically IgA concentrations in the ileum and caecum in different diets (mean±SE, N=12). An asterix above the column indicates a statistical difference to the control.
Figure 2. depicts graphically the caecal microbial community in different feeding groups by illustrating relative proportions of bacteria with differential percentages of guanine plus cytosine (%G+C) in their genomic DNA (mean of four pools).
Figure 3. depicts graphically the correlation between caecal IgA concentration and proportion of microbes with %G+C between 60-64.
Figure 4. depicts graphically the concentration of biogenic amines in the rat caecum (mean±SE, N=12).

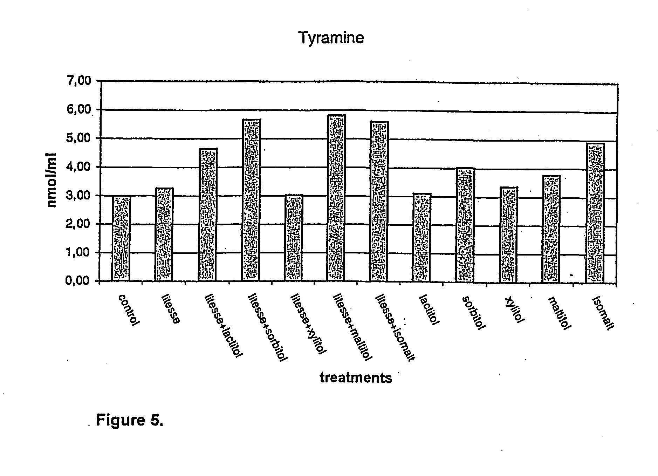

Figure 5. depicts graphically the production of tyramine after 24h fermentation.

### DETAILED DESCRIPTION OF THE INVENTION

The present invention relates to the use of polydextrose in the preparation of a composition for stimulation of the local immune system of the gastrointestinal tract of a mammal, which stimulation is demonstrated by an increased immunoglobulin A concentration in the gut of a mammal. The present invention further includes a potentiation of the immunostimulating effect of polydextrose by the use of polydextrose in combination with a polyol.

In an embodiment of the present invention, an immunostimulating composition is prepared by mixing a dose of polydextrose being effective in increasing the immunoglobulin A concentration in the gut of a mammal, with at least one pharmacologically acceptable carrier.

In another preferred embodiment of the present invention, an immunostimulating composition is prepared by mixing at least one polyol with polydextrose, said polyol being effective to synergistically increase immunoglobulin A in the gut of a mammal.

In another, preferred embodiment, effective amounts of polydextrose and polyol are used to prepare a composition, which reduces the amounts of biogenic amines in the gut of a mammal.

Polydextrose may be used for stimulation of the local immune system of the gastrointestinal tract of a mammal, which is demonstrated by an increased immunoglobulin A concentration in the gut of a mammal.

An effective dose of polydextrose optionally in combination with a polyol may be orally administered to a patient in order to stimulate the immune system in the gut.

In one embodiment of the invention the polydextrose is added to a mammalian food or feed.

In another, preferred embodiment, an effective dose of polydextrose is used in the preparation of a composition for stimulating the immune response in the intestinal tract.

In another preferred embodiment, the polydextrose is added in combination with a polyol to a mammalian food or feed.

In another, preferred embodiment, effective doses of polydextrose and polyol in combination are used in the preparation of a composition for stimulating the immune response in the intestinal tract

In another, preferred embodiment, polydextrose and polyol are used in amounts effective to cause a significant reduction of biogenic amines in the gut.

In another, preferred embodiment, effective doses of polydextrose and polyol in combination are used to prepare a composition to cause a significant reduction of biogenic amines in the gut.

In the present invention, the use of polydextrose unexpectedly increases the concentration of IgA in the gastrointestinal tract of the mammal without at the same time inducing any increase in inflammatory markers. Thus, the effect of polydextrose seems to be of the desired type of immunostimulation.

The increased concentration of IgA in the digesta of test animals is an indication of improved resistance against pathogens via neutralizing antibodies that prevent adhesion on the intestinal epithelium.

The use of polyols in combination with polydextrose synergistically increases the immunostimulating effect of polydextrose even though the polyols alone have no marked effect on the IgA concentration. Thus, the synergistic effect e.g. for lactitol has been found to be about ten-fold when compared to a control diet.

In connection with the present invention, it was noted that polyols significantly increase the relative production of butyrate compared to a control diet. Butyrate, as such is considered as beneficial for the intestine as it is an important energy source for colonocytes regulating cell growth and differentiation. Butyrate is also an interesting volatile fatty acid in terms of reducing colon cancer risk.

Without wishing to be bound by any theory, the present inventors believe that the synergistic effect of polydextrose and polyol may be caused by a combination of two different effects which improve the function of the intestinal immune cells. Thus, polydextrose activates intestinal antibody producing cells, while polyol fermentation offers energy in the form of increased levels of butyrate for the gut immune cells.

A large variety of polyols have been tested for their fermentation pattern in a simulated gut fermentation. All the tested polyols significantly increased the production of butyric acid compared to a control lacking polyol.

The use of polydextrose and polyol also decreases the total biogenic amines in the digesta. Biogenic amines are end products of the bacterial metabolism of proteins and some of them have toxic properties. A reduction of total biogenic amines reduces the risk of colon cancer and suppresses putrefactive fermentation in the gut. Polydextrose and polyol thus improves the function of the gut and reduces the health risks and increases the well-being of the mammal in question.

Polyols are known for having a more or less marked laxative effect which commercially limits the use of polyols in edible products. Thus, a product containing more than 10% polyols should be provided with a warning that the product may have a laxative effect. In connection with the present invention it was found that polydextrose significantly suppresses the laxative effect of the polyols and that a combination of polydextrose and polyol unexpectedly lacks a laxative effect. This will enable the use of larger amounts of polyol in foods and feeds without the risk for laxative side effects.

As mentioned above, a combination of polydextrose and polyol decreases the total biogenic amines. However, surprisingly the levels of one amine, tyramine, is contrarily increased by said combination. Without wishing to be bound by any theory, the present inventors believe that the anti-laxative effect of polydextrose may be caused by the increased production of tyramine in the gut. Tyramine is known to be a local hormone inhibiting the motility of the gut. Decreased motility allows proper absorption of solutes and water from the gut. This improves the tolerance for polyols of the gut.

The term "polydextrose" as used herein is a low calorie polymer of glucose that is resistant to digestion by the enzymes in the stomach. It includes polymer products of glucose which are prepared from glucose, maltose, oligomers of glucose or hydrolyzates of starch, or starch which are polymerized by heat treatment in a polycondensation reaction in the presence of an acid e.g. Lewis acid, inorganic or organic acid, including monocarboxylic acid, dicarboxylic acid and polycarboxylic acid, such as, but not limited to the products prepared by the processes described in the following U.S Patents No: 2,436,967, 2,719,179, 4,965,354, 3,766,165, 5,051,500, 5,424,418, 5,378,491, 5,645,647 or 5,773,604, the contents of all of which are herein incorporated by reference.

The term polydextrose also includes those polymer products of glucose prepared by the polycondensation of glucose, maltose, oligomers of glucose or starch hydrolyzates described hereinabove in the presence of a sugar alcohol, e.g., polyol, such as in the reactions described in U.S. Patent No. 3,766,165. Moreover, the term polydextrose includes the glucose polymers, which have been purified by techniques described in the art, including any and all of the following but not limited to (a) neutralization of any acid associated therewith by base addition thereto, or by passing a concentrated aqueous solution of the polydextrose through an adsorbent resin, a weakly basic ion exchange resin, a type II strongly basic ion-exchange resin, mixed bed resin comprising a basic ion exchange resin, or a cation exchange resin, as described in U.S. Patent No: 5,667,593 and 5,645,647, the contents of both of which are incorporated by reference; or (b) decolorizing by contacting the polydextrose with activated carbon or charcoal, by slurrying or by passing the solution through a bed of solid adsorbent or by bleaching with sodium chlorite, hydrogen peroxide and the like; (c) molecular sieving methods, like UF, RO (reverse osmosis), size exclusion, and the like; (d) or enzymatically treated polydextrose or (e) any other art recognized techniques known in the art.

Moreover, the term polydextrose includes hydrogenated polydextrose which, as used herein, includes hydrogenated or reduced polyglucose products prepared by techniques known to one of ordinary skill in the art. Some of the techniques are described in U.S. Patent No: 5,601,863, 5,620,871 and 5,424,418, the contents of which are incorporated by reference.

It is preferred that the polydextrose used is substantially pure. It may be made substantially pure using conventional techniques known to one skilled in the art, such as chromatography, including column chromatography, HPLC, and the like.

It is more preferred that the polydextrose used is at least 80% pure, i.e. at least about 80% of the impurities are removed. More preferably it is at least 85% pure or even more preferably it is at least 90% pure.

The preferred carrier to be mixed with polydextrose to provide an immunostimulatory composition is a polyol.

The term "polyol" means hexitols such as sorbitol and mannitol, and pentitols such as xylitol. The term also includes C4 -polyhydric alcohols such as erythritol or C12 -polyhydric alcohols such as lactitol *or maltitol.* The term polyol composition means a composition of two or more polyols. Such compositions preferably differ markedlyfrom compositions arising in the industrial preparation of polyols such as sorbitol. Preferred are those compositions which comprise at least two polyols having a different number of C atoms, in particular the term means a composition comprising at least one hexitol and at least one pentitol. Especially for the feed products it is advantageous to utilize polyol mixtures deriving from the industrial production of individual pure polyols. Such mixtures include run-offs or mother liquids from the cristallization of lactitol, maltitol, xylitol etc.

Other carriers and vehicles useful in the preparation of the present immunostimulatory composition are edible and/or nutritional ingredients such as lactose, calcium and other minerals, vitamins, sugars and other components generally included into orally administrable compositions.

Polydextrose is an ingredient designed to give the bulk, texture, mouthfeel and functional attributes of caloric sweeteners. A key to the prior art performance of polydextrose is its caloric value of 1 calorie per gram. Thus its is widely used as a calorie-reduced bulking agent in the dietetic food products. Several studies with dietary fibers have shown, that they improve the intestinal function and health of mammals, because they increase fecal bulk, enhance production of short chain fatty acids, lower fecal pH and increase the growth of favourable microflora (mainly bifidobacteria) in the gut. However, also controversial results relating to the effect of the abundance of total bifidobacteria in the gut have been recently reported. Inulin, which selectively stimulates bifidobacteria in the colon, has been recently found to increase some factors, which are associated with the development of colon cancer (A-M. Pajari et al., British J. Nutrition, 8, pp. 635-643, 2000*,* M. Mutanen et al. Carcinogenesis, Vol. 21, No. 6, pp. 1167-1173, 2000*).* In connection with the present invention it has been noted that an abundance of bifidobacteria may cause also less desirable physiological effects such as enteric bacterial diseases and immunosuppression.

The present inventors have surprisingly found that the administration of polydextrose, especially when administered in combination with a polyol, very significantly increased the immune response in the gut. Polydextrose (and polyol) caused a shift in the microbial community in the gut and enhanced the growth of microbes with a positive effect on the gut immune system. Thus polydextrose can be used e.g. for balancing the microbial community in the gut after antibiotic treatments or other disturbances. Furthermore, the studies revealed, that the administration of polydextrose and a polyol also reduced the biogenic amines in caecum, which are known to increase the risk of colon cancer.

In addition to the beneficial synergistic effects of polydextrose and polyol on the intestinal tract, the present inventors found that the combined administration of polydextrose antagonizes the laxative effects of a polyol. As well known, the use of the polyols is often limited by the undesirable laxative side effects.

Furthermore, another beneficial effect of increased IgA production is the prevention of allergies caused by dietary components. While secretion of IgA to the gut lumen modulates dietary antigen presentation to gut the immune cells, the enhanced antibody production may prevent contact between potential dietary allergens and inflammatory cells, and thus alleviate allergic inflammation.

Without wishing to restrict themselves to any specific theory, the present inventors conclude that this reduction of the laxative effect of polyol when combined with polydextrose may be caused by the elevated level of tyramine in the gut, which was found in the experiments. Since, the use of the polyols is often limited by their undesired laxative effects, the present invention provides also a method for long-term safe use of polyols without the undesired laxative side effects.

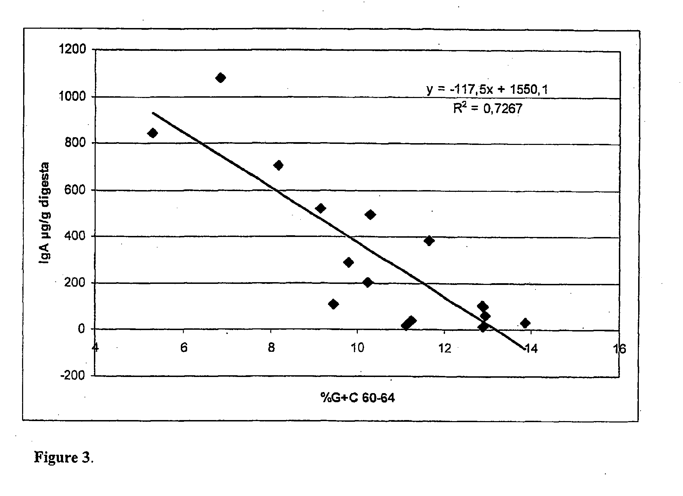

The data of the present invention unexpectedly reveals, that the bacteria with %G+C between 50-69, hence most likely bifidobacteria, correlates negatively with IgA responses (Figure 3.) This area is characteristic of bifidobacteria. It seems that the relative abundance of bifidobacteria went down with a polydextrose-lactitol combined diet, and also with a polydextrose diet. Bifidobacteria as a group are claimed to be beneficial. However, also controversial results relating to effect of the abundance of total bifidobacteria in the gut have been recently reported. In the connection of present invention it has been noted that an abundance of bifidobacteria may cause also less desirable physiological effects such as enteric bacterial diseases and immunosuppression. Microbial %G+C profiling procedure followed steps described Apajalahti et al. (Applied and Environmental Microbiology, Vol. 64. No. 10, pp. 4084-4088, 1998*).* In short, bacteria were separated from digesta samples by differential centrifugation. DNA was then recovered from the bacterial cells by combination of physical, chemical and enzymatic lyses. DNA recovered was then profiled based on the guanine+cytosine content in the DNA of the individual bacteria members of the community. DNAs with different G+C content were separated by CSCI density gradient centrifugation and monitored by pumping the solution through UV flow cell.

The timing of the administration of the polydextrose, as an immune system stimulating agent, either alone or in synergistic effective amounts with a polyol is not critical and can be taken based upon individual needs. The efficient amount of the polydextrose for humans is approximately 1g-100g/day, preferably 5-50g/day. Considering the individual differences

However, a regular daily use is recommended relating to food and feed intake and digestion, polydextrose should cover 0.1-10% of the daily diet, preferably 1-5%, most preferably 2-3%.

In a preferred embodiment of the present invention the polydextrose with an optional polyol is provided in a composition useful for young mammalian animals or babies which are to be weaned. At weaning, the intestinal tract of baby has a very low immune response and stimulation of the function of the gut helps it to stimulate the local immune defense.

The polydextrose, either alone or in synergistic effective amounts with a polyol, is administered to the subject in an amount effective to stimulate the immune response in the intestinal tract of the subject. As used here the term subject refers to animals, especially mammals. Preferred mammals include, but are not limited to man, pet mammalian animals (like dogs, cats, rodents), farm mammal animals (like horses, pigs, cattle, sheep), laboratory mammalian animals and other animals having a similar intestinal tract as those mentioned above. The preferred mammal is man.

The polydextrose is preferably administered to a mammal in a composition which includes polydextrose and an edible carrier or vehicle. The preferred carrier is a polyol which has a synergistic effect on the polydextrose, as described above.

The composition may be prepared in accordance with standard procedures for preparing pharmaceutically, therapeutically or nutritionally acceptable compositions. Thus, the polydextrose may be mixed with the carrier, e.g. with a polyol, and processed further into a dry, semi-dry or liquid product. The polydextrose and polyol may also be granulated to provide a granulate which may be compressed into a tablet as such or with other common excipients and adjuvants, or added to a food or feed to be orally administered to a mammal.

The polydextrose, either alone or in synergistic effective amounts with a polyol can be administered to the subject e.g. by adding in to a food or feed, which is ingested by the subject at or before the meal. Such food include but are not limited to yoghurt, butters, including fruit butters, cream cheese, jellies, jam, preserves, marmalades, cereals, granola bars, confectionary, crackers, dairy desserts, e.g. mousse, frozen foods such as ice cream, sorbet and water ices, infant and baby foods, baked goods, such as cakes, cookies, pastries and other foodstuffs, in beverages such as soft drinks, aqueous solutions, including water, milk, infant and baby beverages and the like; syrups, toppings, sauces and puddings, in salad dressings, mayonnaise, gravy mix, barbecue sauce or other sauce used with meat, fish or poultry, sauces used for pasta and other foods. Polydextrose either alone or in combination with a polyol can be formulated to a capsule, tablet, pill or like by methods known in the art.

The following non-limiting examples further illustrate the invention.

### EXAMPLE 1

Four week old male Wistar rats, divided into a control group and a test group, consisting of 12 rats in each group, were held at a controlled temperature (27±2°C) and 12:12hrs light cycle. The animals had free access to food and water The animals were weighed once a week and their weigh gain was calculated. The control group was fed four weeks with a control (basal) diet and the test group was fed four weeks with the PDX- diet, which consisted of the control (basal) diet supplemented by an immunostimulating amount of polydextrose, as described more in detail below.

### Control (basal) diet

A "Western diet formula" of Djouci and Andrieux (1977), was modified for the trial so that it would be high in cholesterol, salt and protein but low in fiber. Thus, the amounts of energy coming from different sources was calculated as 32% from carbohydrates, 31% from proteins and 36% from fats. The control diet contained (% w/w) potatoes 38.5, minced meat 23.3 wheat bread 8.1, eggs 20.4, butter 1.6 and sugar 8.1. The ingredients were minced, mixed and baked in a steam oven at 200°C for 2-3 hours. The mixture was allowed to cool to room temperature and the following ingredients were mixed (%, w/w) in the diet: vitamin mixture (Altromin International, 1324), 0.87, Cholesterol (Sigma) 0.377 and salt (NaCl) 2.0. The diets were stored in -20°C until used.

The following analyses of the feed were made with bomb calorimetry, inductively coupled plasma emission spectrometry and ion chromatography: heat value 1550cal/g, dry matter 34.2%, ash 0.91%, Na 0.64mg/g, Ca 0.1mg/g, K 3.2mg/g, Mg 0.2mg/g, Cl 1.2mg/g and phosphate 2.5mg/g. The total diet fat content in the diet analysed with AOAC method was 4.8%. The fatty acid profile was analysed by gas chromatography. The basal diet had a total fatty acid content of 3.5%, saturated fatty acid 1.7% and unsaturated fatty acid 1.8%. The ratio for the saturated to unsaturated fatty acids was 0.9.

### PDX-diet

Litesse® (Litesse Ultra, lot no V68011 produced by Danisco Cultor America Inc., which is a processed polydextrose with reduced acidity and bitterness, was added to the basal diet at a 2% (w/w) concentration which was calculated on the basis of human fiber intake recommendations and the energy requirements of rats. The recommendations of fiber intake for adults vary between 25-35g/day or 3g/MJ. In rat the corresponding figures are 120mg/day or 1.47g/day taking the energy consumption difference between rat and man into account, 0.49MJ and 10.47MJ, respectively. Based on the energy value of the basal diet the feed consumption of a rat was approximately 40g/day. The content of the supplemented polydextrose in the diet was then designed to be at a moderate 2% level (20g/kg diet).

### Immunological analyses

IgA was extracted from the ileal and caecal digesta and the concentration of IgA measured from the supernatant with a commercially available ELISA kit. The results were expressed as µg IgA per g digesta (wet weight).

### Results

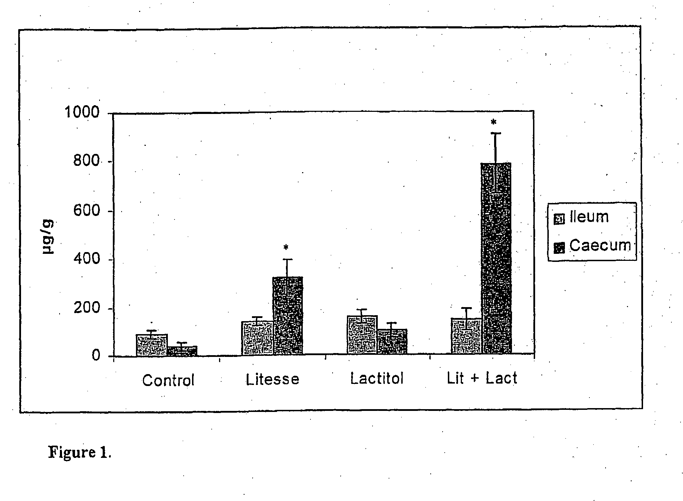

The results showed that the diet with added polydextrose increased several fold the concentration of IgA (+345%, p=0.007), which can be seen in the Figure 1.

### EXAMPLE 2

Four week old male Wistar rats, divided into a control group and three test groups, consisting of 12 rats in each group, were held at a controlled temperature (27±2°C) and 12:12hrs light cycle. The animals had free access to food and water. The animals were weighed once a week and their weight gain was calculated. The control group was fed four weeks with a control (basal) diet and the test group was fed four weeks with the PDX-Polyol-diet, which consisted the control (basal) diet supplemented by an immunostimulating amount of polydextrose, and polyol as described in more detail below.

### Control (basal) diet

The control diet was the same diet as defined in Example 1.

### PDX-Polyol- diet

Litesse® (Litesse Ultra lot no V68011, produced by Danisco Cultor Americ Inc.) and lactitol (N125/22459, produced by Danisco Sweeteners) were added to the basal diet at a 2% (w/w) concentration *of* each, which was calculated on the basis of human fiber intake recommendations and the energy requirements of rats.

### Sampling

Digesta samples were taken from ileum, caecum and colon. Dry matter and IgA measurements and microbial counts and %G+C were taken from individual ileal and caecal digesta samples. The samples were stored in -20°C until used. The pH was measured from the caecum *in situ.* Intestinal tissue samples for histological and immunohistological analysis were taken from the distal ileum an distal caecum.

### Immunological analyses

IgA was extracted from the ileal digesta and caecal and the concentration of IgA measured from the supernatant with a commercially available ELISA kit. The results were expressed as µg IgA per g digesta (wet weight).

### Microbial analyses

In order to determine the total number of microbes present in the caecal digesta samples, the cells were separated from the collected digesta by a washing procedure (Apajalahti et al. Applied and Environmental Microbiology, Vol. 64. No. 10, pp. 4084-4088, 1998*).* For counting, a sample of separated cells from each caecum digesta pool (four per treatment, 16 altogether) was appropriately diluted and the cells stained with a fluorescent, nucleic acid binding dye (Molecular Probes) having absorption at wavelength of 490nm and emission at 515nm.

After staining, the amount of events per fixed time interval was determined by running the sample into FACSCalibur (Becton, Dickinson and Company) flow cytometer with parameters adjusted suitable for counting microbes. The cells were quantified by using BD TrueCount™ Tubes including a certain amount of fixed sized fluorescent beads. During analysis, the absolute number of cells in the sample could be determined by comparing cellular events to bead events. The results were reported as cells/g wet caecal digesta.

Microbial %G+C profiling procedure followed steps described Apajalahti et al. (Applied and Environmental Microbiology, Vol. 64. No. 10, pp. 4084-4088, 1998*).*

### Results

When polydextrose was a sole added supplement in the diet (see Example 1), the concentration of IgA was increased several fold, +345% (p=0.007), when compared to the control group. A significant synergistic effect was achieved when a combination of polydextrose and lactitol was added into the diet, the increase of the IgA level was as high as +996% (p=0.0001), when compared to the control group. The results are shown in Figure 1.

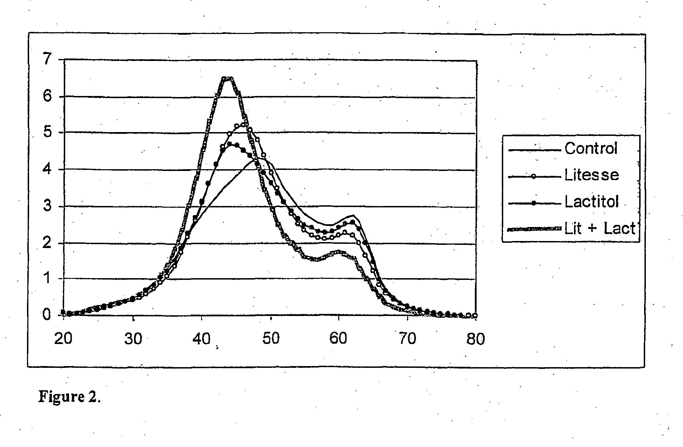

The structure of the total microbial community was analysed by a technique known as %G+C profiling. It measures the composition of the total microbial community DNA isolated from digesta samples. In this study it was found that the relative abundance of different bacterial groups changed when polydextrose, or polydextrose in combination with lactitol was added to the diet. The most pronounced change was observed in the relative abundance of bacteria with %G+C between 35 and 45. The other %G+C range, where clear changes took place, was around 60% G+C (Figure 2).

### EXAMPLE 3

Four week old male Wistar rats, divided into a control group and three test groups, consisting of 12 rats in each group, were held at a controlled temperature (27±2°C) and 12:12hrs light cycle. The animals had free access to food and water. The animals were weighed once a week and their weight gain was calculated. The control group was fed four weeks with a control (basal) diet and the test group was fed four weeks with the PDX-Polyol-diet, which consisted the control (basal) diet supplemented by an immunostimulating amount of polydextrose, and polyol as described in more detail below.

### Control (basal) diet and PDX-Polyol- diet

The control diet was the same diet as defined in Example 1. and the PDX-Polyol-diet was the same as defined in Example 2.

### Sampling

The digesta samples from caecum and colon were divided into pools (3 animals per pool) for the analysis of biogenic amines. The samples were stored in -20°C until analysed. PH was measured from the caecum in situ.

### Analyses biogenic amines from digesta

Biogenic amines were determined from caecal digesta samples. Heptylamine was added to the samples as an internal standard and the biogenic amines were extracted with 0.4M perchloric acid. After centrifugation the biogenic amines in the supernatant were derivatised with dansyl chloride. The derivatives were separated on a C18 reversed phase column using mixture of ammonium acetate and acetonitrile as eluent, and detected with fluorescence detector.

### Results

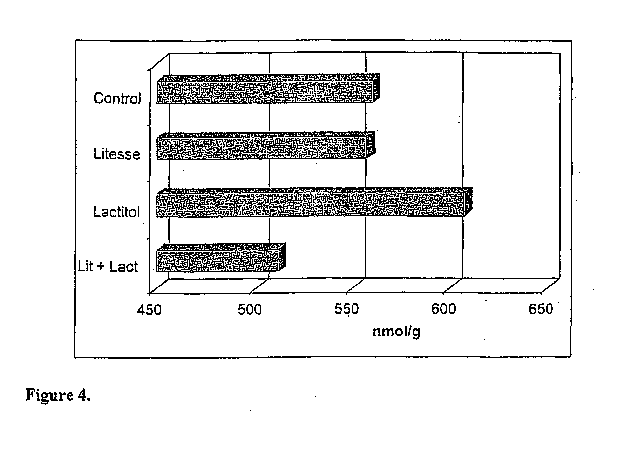

The combined use of polydextrose and lactitol caused a reduction of 11% (p00.03) in the sum of all biogenic amines (Figure 4). This indicates a reduction of putrefaction of dietary proteins and also indicates that the relative abundance of putrefactive bacteria (e.g. clostridia), generally considered harmful, is decreased.

It is significant to note that the level of tyramine is substantially affected by polydextrose and polyols. Thus polydextrose increased the amount of tyramine compared to a control diet. Lactitol, on the other hand, reduced the amount of tyramine. When polydextrose was used in combination with lactitol, the result was surprisingly an increase in tyramine by 188% compared to the control diet.

Without wishing to be bound by any theory, the inventors are of the opinion that the higher tyramine level could explain the suppression of the laxative effect of lactitol when it is administered in combination with polydextrose, since it is known that tyramine exhibits inhibition of colonic motility.

### EXAMPLE 4

A batch fermentation test was performed in vitro in order to confirm the tests performed in vivo in Examples 1 to 3.

The test was based on a 24h fermentation experiment performed with human fecal samples. Fecal samples were incubated in 24-hour batch cultures at 37°C at pH 6.9 under anaerobic conditions containing a buffer medium (mineral salt, trace elements, vitamines). The medium was supplemented with 10% extract of caecal digesta from pig. Polydextrose, polyols and their combinations were added at the level of 1% each into the test cultures.

### Results

All polyols increased the relative and absolute production of butyric acid. The fermentation pattern is similar for all the polyols and for the combinations of polyols and polydextrose. The production of butyric acid was significantly increased in all of the test samples compared to the control (Table 1.)

The fermentation of polyols increases the production of butyrate, which is a source of energy for the gut immune cells. The very significant potentiation of the immunostimulating effect of polydextrose when polydextrose was administered in combination with a polyol (Example 2) may be caused by the increased energy in the form of butyrate, which on the other hand leads to the increased production of immunoglobulin A by the gut immune cells.

### EXAMPLE 5

The in vivo test of Example 2 and the in vitro fermentation of Example 3 were repeated and the level of tyramine was measured.

**Table 1**

| Production of SCFA (%) after 24 h fermentation. | | | | | | | | | | | | |
|---|---|---|---|---|---|---|---|---|---|---|---|---|
| | Control | Litesse | Litesse+ lactitol | Litesse+ sorbitol | Litesse+ xylitol | Litesse+ maltitol | Litesse+ isomalt | lactitol | Sorbitol | Xylitol | Maltitol | Isomalt |
| | (%) | (%) | (%) | (%) | (%) | (%) | (%) | (%) | (%) | (%) | (%) | (%) |
| Acetic acid | 66.1 | 67.1 | 65.2 | 52.0 | 29.9 | 57.2 | 61.9 | 66.2 | 55.2 | 45.8 | 57.9 | 64.0 |
| Propionic acid | 12.8 | 12.8 | 9.0 | 12.4 | 12.7 | 10.9 | 10.6 | 9.3 | 11.4 | 12.6 | 12.1 | 12.8 |
| Isobutyric acid | 1.3 | 0.6 | 0.3 | 0.4 | 0.5 | 0.4 | 0.4 | 0.4 | 0.5 | 0.6 | 0.4 | 0.4 |
| Butyric acid | 15.1 | 16.9 | 23.3 | 33.8 | 55.0 | 29.4 | 22.9 | 22.8 | 31.1 | 38.6 | 27.9 | 21.3 |
| 2-met.butyric acid | 0.7 | 0.3 | 0.2 | 0.2 | 0.3 | 0.2 | 0.2 | 0.2 | 0.2 | 0.3 | 0.2 | 0.2 |
| Isovaleric acid | 0.8 | 0.4 | 0.2 | 0.2 | 0.3 | 0.2 | 0.2 | 0.2 | 0.3 | 0.3 | 0.2 | 0.2 |
| Lactic acid | | | 1.1 | | | 0.8 | 2.8 | | | | | |
| Valeric acid | 3.1 | 1.9 | 0.8 | 1.1 | 1.5 | 1.0 | 1.0 | 0.9 | 1.4 | 1.9 | 1.2 | 1.2 |
| Total SCFA (mmol/l) | 82.4 | 120.0 | 160.2 | 141.3 | 138.8 | 172.6 | 162.1 | 153.6 | 122.4 | 191.6 | 157.0 | 158.6 |

The in vivo test showed a significant increase of the tyramine level with the feeding of polydextrose and lactitol. At the same time a significant suppression of the laxative effect which was caused by lactitol alone was observed in the test animals.

The in vitro fermentation with several polyols confirmed that a similar increased production of tyramine appears with a combination of polydextrose and lactitol, sorbitol, maltitol and isomalt. Thus, these polyols produced tyramine in the same way as lactitol in combination with polydextrose.

It seems that the combination of polydextrose and polyol increases the level of tyramine and that this effect may account for the suppressed laxative influence observed in the in vivo test.

### EXAMPLE 6 Yoghurt product

Pectin (Grindsted Pectin YF 310) 0.70% and crystalline lactitol 1.0% were blended dry and dissolved in water 11.0%, that had been heated to 80-85°C. Raspberries (frozen) 50.0% polydextrose (Litesse® Ultra™, produced by Danisco Cultor America Inc.) 18.80% and crystalline lactitol 17.80% were heated to boil and after that the mixture of pectin and lactitol was added while agitating well. A calcium slurry was made by dissolving a calcium salt (Calcium lactate 5 H₂O) 0.296% in hot water 5.0%, and then it was added to the fruit mass, while agitating well. The mixture was then evaporated until the desired content was reached. pH was adjusted to 3.9 using sodium citrate solution, and preservatives (K-sorbate 20%w/v, 0.25%) were added. For filling the mixture was cooled to temperature of 40°C. The mixture was dosed into a youghurt at a final dosage of 15-20%. Ingestion of one to two youghurts per day increased the concentration of IgA in the gut. The percentages are calculated on fresh weight basis.

### EXAMPLE 7 Polydextrose hard candy centre filled with xylitol and polydextrose.

The ingredients for a hard candy filling, crystalline xylitol 30.1%, polydextrose (Litesse® Ultra, produced by Danisco Cultor America Inc.) 45.1% and water 24.8% were mixed together and then heated to 90.0% Dry Substance (Approx. 119°C.) The resulting syrup was transferred to a dosing pump hopper. The ingredients for a hard candy shell, polydextrose 97.0% and crystalline xylitol 3.0% were mixed together, water was added at 25.0% and then the mix was heated to 135°C. Peppermint oil q.s, aspartame 0.10% and acesulfame K 0.05% were added to the candy mass when it had cooled to <100°C and the mass was then tempered until a suitable texture for stamping. The candy mass was centre filled with the xylitol/polydextrose filling via a dosing pump attached to a central pipe through the candy mass. The candies were left to cool before wrapping. The candies were sugar-free products suitable for dietetics and increasing the level of IgA in the gut. The percentages are calculated on fresh weight basis.

### EXAMPLE 8 Toffee topping

Polydextrose (Litesse® II, produced by Danisco Cultor America Inc.) 13.9% and crystalline lactitol 14.4% were blended dry. The blend of polydextrose and lactitol, evaporated milk 11.8%, maltitol syrup 49.8%, salt 0.5%, GMS 0.4%, lecithin 0.2% and butterfat 8.8% were pre-mixed at 65°C for 20 minutes. The mixture was then cooked to 124°C. Vanillin 0.1%, aspartame 0.1% and flavour were added. The mixture was used to top countline bars providing an increased level of IgA in the gastrointestinal tract. The percentages are calculated on fresh weight basis.

### EXAMPLE 9 Plain chocolate

Cocoa liquor 42.0% and cocoa butter 11.4% were melted. Anhydrous crystalline (or anhydrous crystalline milled) lactitol 32.0% and polydextrose (Litesse® Ultra, produced by Danisco Cultor America Inc.) 13.8% were mixed with cocoa liquor, part of cocoa butter and vanillin. The mixture was refined to required particle size, conched at 60°C for up to 24 hours and the remainder of the cocoa butter was added. Lecithin and aspartame were added one hour before end of conching. The chocolate was sugar-free and suitable for diabetics and for increasing IgA in the gut. The percentages are calculated on fresh weight basis.

### EXAMPLE 10 Madeira cake

Whole fresh eggs 18.03%, and water 15.72% were placed in the bowl of a planetary mixer. Crystalline lactitol 20.73%, polydextrose (Litesse® II, produced by Danisco Cultor America Inc.) 5.21%, heat-treated cake flour 22.53%, skimmed milk powder 1.60%, spray dried egg albumin 1.10%, baking powder 0.90%, salt 0.60%, acesulfame K 0.06% and high ratio fat 13.52% were then added, mixed on speed 1 for 30 seconds, and scraped down. After that the mixture was mixed on speed 2 for 1.5 minutes and scraped down. Once again, mixed on speed 2 for a further 1.5 minutes until a relative density of 0.7-0.75 was achieved. The dough was deposited into paper cases in 11b-cake tins and baked for 30 minutes at 200°C. The cake was useful for increasing the IgA in the gut without any laxative effect. The percentages are calculated on fresh weight basis.

Especially, the very significant synergistic effect of the two substances was clearly shown by the experiments.

The preferred ratios described hereinabove with respect to polydextrose and lactitol are also applicable to the mixtures of the polydextrose with other polyols.

Unless indicated to the contrary, the percentages are weight percentages. Moreover, the weights provided are the dry weights, i.e., excluding the weight of the carrier, which may be present.

The above preferred embodiments and examples are given to illustrate the scope and spirit of the present invention. These embodiments and examples will make apparent to those skilled in the art other embodiments and examples. These other embodiments and examples are within the contemplation of the present invention.

## Claims

1. The use of polydextrose in the preparation of a composition for stimulating the immune system of the gastrointestinal tract of a mammal, comprising mixing at least one pharmacologically acceptable carrier or vehicle with polydextrose, said polydextrose being effective in increasing the immunoglobulin A (IgA) concentration in the gut of said mammal.

2. The use according to claim 1 wherein said carrier comprises a polyol.

3. The use according to claim 2 comprising mixing polydextrose with a polyol, said polyol being effective to synergistically increase the immunoglobulin A (IgA) concentration in the gut of said mammal.

4. The use according to claim 3, comprising mixing polydextrose with lactitol, said lactitol being effective to synergistically increase the immunoglobulin A (IgA) concentration increasing effect of said polydextrose.

5. The use of according to claim 2, wherein said polydextrose and polyol are additionally effective in reducing the amounts of biogenic amines in the gut.

6. The use of according to claim 2, wherein said polydextrose is additionally effective in suppressing the laxative effect of said polyol.

7. The use according to claim 1 or 2 wherein said polydextrose is additionally effective in balancing or normalizing the gut microbial community.

8. The use according to any one of claims 1 to 7, wherein said mammalian is selected from the group comprising human beings, mammalian pet animals, mammalian farm animals, mammalian laboratory animals, mammalian zoo animals.

9. The use of claim 8 wherein said mammal is a young mammal at the age of weaning.

10. The use according to claim 1 or 2 wherein said composition is prepared in the form of an orally administrable preparation selected from the group comprising a dry, semidry or liquid food product, a tablet, a pill, a chewing gum or tablet, a powder, a spray, a syrup, a sugar substitute, a candy or sweet, a dairy product, a frozen dairy product, a pet food, an animal feed, and the like.

11. The use according to claim 1 or 2, wherein said polydextrose is hydrogenated polydextrose.

12. The use according to claim 1 or 2, wherein said polydextrose is purified.

13. The use according to claim 2, wherein said polyol is selected from group comprising lactitol, xylitol, maltitol, sorbitol, isomalt.

14. The use according to claim 2, wherein said polyol is lactitol or xylitol.

15. The use according to claim 14, wherein said polyol is lactitol.

16. The use according to claim 1, wherein polydextrose is incorporated into a composition to be administered orally.

17. The use according to claim 2, wherein polydextrose in combination with polyol is incorporated into a composition to be administered orally.

18. The use according to claim 17, wherein polydextrose in combination with lactitol is incorporated into composition to be administered orally.

19. The use according to claim 2, wherein the weight ratio of polyol to polydextrose ranges from about 1:10 to 10:1:

20. The use according to claim 19, wherein the weight ratio ranges from 1:5 to about 5:1

21. The use according to claim 20, wherein the weight ratio is about 1:1.

22. The use according to claim 16, wherein the polydextrose is added to a food product in effective amounts to stimulate the immune system of the gastrointestinal tract of a mammal, when the food containing the same is administered to the mammal.

23. The use according to claim 17, wherein polyol and polydextrose are added to a food product in synergistic effective amounts to stimulate the immune system of the gastrointestinal tract of a mammal, when the food containing the same is administered to the mammal.

24. The use according to claim 17, wherein the polyol and polydextrose are added to a food product in synergistic effective amounts to reduce the biogenic amines in the caecum of a mammal, when the food containing the same is administered to the mammal.

25. The use according to claim 16 or 17, wherein polydextrose is added to said composition in an amount effective to balance or normalize the microbial community of an a mammal after an antibiotic treatment or other disturbance of the gut.

26. The use according to claim 1, wherein the polydextrose is non-hydrogenated polydextrose, hydrogenated polydextrose or non- hydrogenated polydextrose or hydrogenated polydextrose, which has been subject to purification or a mixture thereof.

27. An orally administrable composition comprising at least one pharmacologically acceptable carrier or vehicle and polydextrose in an amount sufficient to increase the concentration of immunoglobulin A (IgA) in the gut of a mammal for use in stimulating the immune system of the gastrointestinal tract of said mammal.

28. The composition of claim 27 comprising polydextrose and polyol, said polyol providing a synergistic potentiation of the immunostimulation of polydextrose.

29. The composition of claim 27 or 28 wherein said polydextrose is purified polydextrose and said polyol is selected from the group consisting of lactitol, sorbitol, maltitol, xylitol and isomalt

30. The composition of claim 27 or 28 which is a nutritional food or feed product.

31. The composition of claim 27 or 28 which is in the form of a dry, semidry or liquid product for young mammals at weaning.

## Patentansprüche

1. Verwendung von Polydextrose in der Herstellung einer Zusammensetzung zum Stimulieren des Immunsystems des Magen-Darm-Trakts eines Säugers, wobei man mindestens einen pharmakologisch unbedenklichen Träger oder mindestens ein pharmakologisch unbedenkliches Konstituens mit Polydextrose vermischt, wobei die Polydextrose dahingehend wirkt, dass sie die Konzentration an Immunglobulin A (IgA) im Darm des Säugers erhöht.

2. Verwendung nach Anspruch 1, wobei der Träger ein Polyol umfasst.

3. Verwendung nach Anspruch 2, wobei man Polydextrose mit einem Polyol vermischt, wobei das Polyol dahingehend wirksam ist, dass es die Konzentration an Immunglobulin A (IgA) im Darm des Säugers synergistisch erhöht.

4. Verwendung nach Anspruch 3, wobei man Polydextrose mit Lactid vermischt, wobei das Lactid dahingehend wirksam ist, dass es die Konzentration an Immunglobulin A (IgA) synergistisch erhöht, wodurch die Wirkung der Polydextrose erhöht wird.

5. Verwendung nach Anspruch 2, wobei die Polydextrose und das Polyol zusätzlich dahingehend wirksam sind, dass sie die Mengen an biogenen Aminen im Darm reduzieren.

6. Verwendung nach Anspruch 2, wobei die Polydextrose zusätzlich dahingehend wirksam ist, dass sie die abführende Wirkung des Polyols unterdrückt.

7. Verwendung nach Anspruch 1 oder 2, wobei die Polydextrose zusätzlich dahingehend wirksam ist, dass sie die Mikroorganismenflora im Darm ausgleicht oder normalisiert.

8. Verwendung nach einem der Ansprüche 1 bis 7, wobei der Säuger aus der Gruppe umfassend Menschen, Hobbytier-Säuger, Nutztier-Säuger, Labortier-Säuger und Zootier-Säuger ausgewählt ist.

9. Verwendung nach Anspruch 8, wobei es sich bei dem Säuger um einen jungen Säuger im Absetzalter handelt.

10. Verwendung nach Anspruch 1 oder 2, wobei die Zusammensetzung in Form eines oral verabreichbaren Präparats, ausgewählt aus der Gruppe umfassend ein trockenes, halbtrockenes oder flüssiges Nährprodukt, eine Tablette, eine Pille, einen Kaugummi oder eine Kautablette, ein Pulver, einen Spray, einen Sirup, einen Zuckerersatz, eine Süßware oder Zuckerware, ein Milchprodukt, ein gefrorenes Milchprodukt, ein Hobbytierfutter, ein Tierfutter und dergleichen, hergestellt wird.

11. Verwendung nach Anspruch 1 oder 2, wobei es sich bei der Polydextrose um hydrierte Polydextrose handelt.

12. Verwendung nach Anspruch 1 oder 2, wobei die Polydextrose gereinigt ist.

13. Verwendung nach Anspruch 2, wobei das Polyol aus der Gruppe umfassend Lactid, Xylit, Maltit, Sorbit und Isomalt ausgewählt ist.

14. Verwendung nach Anspruch 2, wobei es sich bei dem Polyol um Lactid oder Xylit handelt.

15. Verwendung nach Anspruch 14, wobei es sich bei dem Polyol um Lactid handelt.

16. Verwendung nach Anspruch 1, wobei Polydextrose in eine Zusammensetzung zur oralen Verabreichung eingearbeitet wird.

17. Verwendung nach Anspruch 2, wobei Polydextrose in Kombination mit Polyol in eine Zusammensetzung zur oralen Verabreichung eingearbeitet wird.

18. Verwendung nach Anspruch 17, wobei Polydextrose in Kombination mit Lactid in eine Zusammensetzung zur oralen Verabreichung eingearbeitet wird.

19. Verwendung nach Anspruch 2, wobei das Gewichtsverhältnis von Polyol zu Polydextrose im Bereich von ungefähr 1:10 bis 10:1 liegt.

20. Verwendung nach Anspruch 19, wobei das Gewichtsverhältnis im Bereich von 1:5 bis ungefähr 5:1 liegt.

21. Verwendung nach Anspruch 20, wobei das Gewichtsverhältnis ungefähr 1:1 beträgt.

22. Verwendung nach Anspruch 16, wobei die Polydextrose einem Nährprodukt in Mengen zugesetzt wird, die dahingehend wirksam sind, dass sie das Immunsystem des Magen-Darm-Trakts eines Säugers stimulieren, wenn die Nahrung, die jene enthält, dem Säuger verabreicht wird.

23. Verwendung nach Anspruch 17, wobei Polyol und Polydextrose einem Nährprodukt in Mengen zugesetzt werden, die dahingehend synergistisch wirksam sind, dass sie das Immunsystem des Magen-Darm-Trakts eines Säugers stimulieren, wenn die Nahrung, die jene enthält, dem Säuger verabreicht wird.

24. Verwendung nach Anspruch 17, wobei das Polyol und die Polydextrose einem Nährprodukt in Mengen zugesetzt werden, die dahingehend synergistisch wirksam sind, dass sie die biogenen Amine im Blinddarm eines Säugers reduzieren, wenn die Nahrung, die jene enthält, dem Säuger verabreicht wird.

25. Verwendung nach Anspruch 16 oder 17, wobei Polydextrose der Zusammensetzung in einer Menge zugesetzt wird, die dahingehend wirksam ist, dass sie die Mikroorganismenflora eines Säugers nach Antibiotikabehandlung oder sonstiger Störung des Darms ausgleicht oder normalisiert.

26. Verwendung nach Anspruch 1, wobei es sich bei der Polydextrose um nichthydrierte Polydextrose, hydrierte Polydextrose oder nichthydrierte Polydextrose oder hydrierte Polydextrose, die einer Reinigung unterzogen worden ist, oder eine Mischung davon handelt.

27. Zusammensetzung zur oralen Verabreichung, umfassend mindestens einen pharmakologisch unbedenklichen Träger bzw. mindestens ein pharmakologisch unbedenkliches Konstituens sowie Polydextrose in einer Menge, die ausreicht, um die Konzentration an Immunglobulin A (IgA) im Darm eines Säugers zu erhöhen, zur Verwendung bei der Stimulierung des Immunsystems des Magen-Darm-Trakts des Säugers.

28. Zusammensetzung nach Anspruch 27, die Polydextrose und Polyol umfasst, wobei das Polyol eine synergistische Wirkungssteigerung der Immunstimulation der Polydextrose bereitstellt.

29. Zusammensetzung nach Anspruch 27 oder 28, wobei es sich bei der Polydextrose um gereinigte Polydextrose handelt und das Polyol aus der Gruppe bestehend aus Lactid, Sorbit, Maltit, Xylit und Isomalt ausgewählt ist.

30. Zusammensetzung nach Anspruch 27 oder 28, bei der es sich um ein diätetisches Nahrungs- oder Futtermittelprodukt handelt.

31. Zusammensetzung nach Anspruch 27 oder 28, die in Form eines trockenen, halbtrockenen oder flüssigen Produkts für junge Säuger beim Absetzen vorliegt.

## Revendications

1. Utilisation de polydextrose dans la préparation d'une composition destinée à stimuler le système immunitaire du tractus gastro-intestinal d'un mammifère, comprenant le mélange d'au moins un support ou véhicule pharmacologiquement acceptable avec du polydextrose, ledit polydextrose étant efficace pour augmenter la concentration en immunoglobuline A (IgA) dans les intestins dudit mammifère.

2. Utilisation selon la revendication 1, dans laquelle ledit support comprend un polyol.

3. Utilisation selon la revendication 2, comprenant le mélange de polydextrose avec un polyol, ledit polyol étant efficace pour augmenter de manière synergique la concentration en immunoglobuline A (IgA) dans les intestins dudit mammifère.

4. Utilisation selon la revendication 3, comprenant le mélange du polydextrose avec du lactitol, ledit lactitol étant efficace pour augmenter de manière synergique la concentration en immunoglobuline A (IgA) augmentant l'effet dudit polydextrose.

5. Utilisation selon la revendication 2, dans laquelle lesdits polydextrose et polyol sont en outre efficaces pour réduire les quantités d'amines biogéniques dans les intestins.

6. Utilisation selon la revendication 2, dans laquelle ledit polydextrose est en outre efficace pour supprimer l'effet laxatif dudit polyol.

7. Utilisation selon la revendication 1 ou 2, dans laquelle ledit polydextrose est en outre efficace pour équilibrer ou normaliser la communauté microbienne intestinale.

8. Utilisation selon l'une quelconque des revendications 1 à 7, dans laquelle ledit mammifère est choisi dans le groupe constitué par les êtres humains, les animaux familiers mammaliens, les animaux de ferme mammaliens, les animaux de laboratoire mammaliens, les animaux de zoo mammaliens.

9. Utilisation selon la revendication 8, dans laquelle ledit mammifère est un jeune mammifère à l'âge du sevrage.

10. Utilisation selon la revendication 1 ou 2, dans laquelle ladite composition est préparée sous la forme d'une préparation administrable par voie orale choisie dans le groupe constitué par un produit alimentaire sec, semi-sec ou liquide, un comprimé, une pilule, un chewing-gum ou un cachet, une poudre, un spray, un sirop, un succédané de sucre, une confiserie ou un bonbon, un produit laitier, un produit laitier glacé, un aliment pour animaux familiers, un aliment pour animaux, et similaires.

11. Utilisation selon la revendication 1 ou 2, dans laquelle ledit polydextrose est du polydextrose hydrogéné.

12. Utilisation selon la revendication 1 ou 2, dans laquelle ledit polydextrose est purifié.

13. Utilisation selon la revendication 2, dans laquelle ledit polyol est choisi dans le groupe constitué par le lactitol, le xylitol, le maltitol, le sorbitol, l'isomalt.

14. Utilisation selon la revendication 2, dans laquelle ledit polyol est le lactitol ou le xylitol

15. Utilisation selon la revendication 14, dans laquelle ledit polyol est le lactitol.

16. Utilisation selon la revendication 1, dans laquelle du polydextrose est incorporé dans une composition devant être administrée par voie orale.

17. Utilisation selon la revendication 2, dans laquelle du polydextrose en combinaison avec du polyol est incorporé dans une composition devant être administrée par voie orale.

18. Utilisation selon la revendication 17, dans laquelle du polydextrose en combinaison avec du lactitol est incorporé dans une composition devant être administrée par voie orale.

19. Utilisation selon la revendication 2, dans laquelle le rapport pondéral du polyol au polydextrose va d'environ 1:10 à 10:1.

20. Utilisation selon la revendication 19, dans laquelle le rapport pondéral va de 1:5 à environ 5:1.

21. Utilisation selon la revendication 20, dans laquelle le rapport pondéral est d'environ 1:1.

22. Utilisation selon la revendication 16, dans laquelle le polydextrose est ajouté à un produit alimentaire selon des quantités efficaces pour stimuler le système immunitaire du tractus gastro-intestinal d'un mammifère, lorsque l'aliment contenant celui-ci est administré au mammifère.

23. Utilisation selon la revendication 17, dans laquelle du polyol et du polydextrose sont ajoutés à un produit alimentaire selon des quantités efficaces synergiques pour stimuler le système immunitaire du tractus gastro-intestinal d'un mammifère, lorsque l'aliment contenant ceux-ci est administré au mammifère.

24. Utilisation selon la revendication 17, dans laquelle du polyol et du polydextrose sont ajoutés à un produit alimentaire selon des quantités efficaces synergiques pour réduire les amines biogéniques dans le caecum d'un mammifère, lorsque l'aliment contenant ceux-ci est administré au mammifère.

25. Utilisation selon la revendication 16 ou 17, dans laquelle le polydextrose est ajouté à ladite composition selon une quantité efficace pour équilibrer ou normaliser la communauté microbienne d'un mammifère après un traitement antibiotique ou une autre perturbation des intestins.

26. Utilisation selon la revendication 1, dans laquelle le polydextrose est du polydextrose non hydrogéné, du polydextrose hydrogéné, ou du polydextrose non hydrogéné ou du polydextrose hydrogéné, ayant été soumis à une purification, ou un mélange de ceux-ci.

27. Composition administrable par voie orale, comprenant au moins un support ou véhicule pharmacologiquement acceptable et du polydextrose selon une quantité suffisante pour augmenter la concentration en immunoglobuline A (IgA) dans les intestins d'un mammifère, pour une utilisation dans la stimulation du système immunitaire du tractus gastro-intestinal dudit mammifère.

28. Composition selon la revendication 27, comprenant du polydextrose et du polyol, ledit polyol fournissant une potentialisation synergique du l'immuno-stimulation du polydextrose.

29. Composition selon la revendication 27 ou 28, dans laquelle ledit polydextrose est du polydextrose purifié et ledit polyol est choisi dans le groupe constitué par le lactitol, le sorbitol, le maltitol, le xylitol et l'isomalt.

30. Composition selon la revendication 27 ou 28, qui est un produit d'aliment ou d'aliment pour animaux nutritionnel.

31. Composition selon la revendication 27 ou 28, qui se trouve sous la forme d'un produit sec, semi-sec ou liquide pour de jeunes mammifères lors du sevrage.
